Europäisches Patentamt

European Patent Office

Office européen des brevets

(11) Publication number: **0 222 332**
A2

(12) **EUROPEAN PATENT APPLICATION**

(21) Application number: 86115420.1

(22) Date of filing: 06.11.86

(51) Int. Cl.⁴: **C 12 N 15/00**
C 07 K 15/04, C 07 K 17/14
C 12 Q 1/68, C 12 N 1/20
C 12 P 21/02

(30) Priority: 08.11.85 US 796372

(43) Date of publication of application:
20.05.87 Bulletin 87/21

(84) Designated Contracting States:
BE CH DE FR GB IT LI NL

(71) Applicant: F. HOFFMANN-LA ROCHE & CO.
Aktiengesellschaft

CH-4002 Basel(CH)

(72) Inventor: Furuichi, Yasuhiro
3-24-9, Terabun
Kamakura-shi Kanagawa-ken(JP)

(72) Inventor: Richardson, Michael A.
Nagase Bldg., Rm. 404 100 Kamigo-cho
Totsuka-ku Yokohama-shi(JP)

(74) Representative: Lederer, Franz, Dr. et al,
Patentanwält Dr. Franz Lederer Lucile Grahnstrasse 22
D-8000 München 80(DE)

(54) Recombinant sigma NS protein.

(57) The present invention relates to the synthesis by application of recombinant DNA technology of recombinant reovirus nonstructural protein sigma NS and its use as a non-specific binding agent for single stranded ssRNA's. This property of sigma NS protein can be employed when it is used as a reagent in protecting unstable RNA during extraction processes and more significantly as a reagent for the consentration of ssRNA samples for use in hybridization probe assays.

F I G. 1

Basel / Schweiz

6. Nov. 1986

**0222332**

RAN 4100/59

## Recombinant Sigma NS Protein

This invention relates to the synthesis by application of recombinant DNA technology of recombinant reovirus non-structural protein sigma ($\sigma$) NS and its use as a non--specific binding agent for single stranded (ss) RNA's. This property of $\sigma$ NS protein can be employed when it is used as a reagent in protecting unstable RNA during extraction processes and more significantly as a reagent for the concentration of ssRNA samples for use in hybridization probe assays.

The reovirus genome consists of 10 segments of double--stranded (ds) RNA. The segments are transcribed by virus--associated RNA polymerase to form capped mRNAs which also function as templates for a putative replicase in virus--infected cells. Each ds RNA segment codes for at least one protein. Two of these proteins, $\mu_{NS}$ and $\sigma_{NS}$, encoded by genomic segments M3 and S3, respectively, are found only in infected cells. While the function of these two nonstructural proteins is unknown, there is some evidence to suggest that $\sigma_{NS}$ protein may act in the selection and condensation of the 10 different ssRNAs into precursor subviral particles in preparation for dsRNA synthesis.

It is thus known in the art that $\sigma_{NS}$ protein has the capacity to bind ssRNAs as part of the putative viral replication system in infected cells (Huismans and Joklik, Virology 70, 411-424 [1976]). In addition, virus-specific particles sedimenting at 13-19S which are composed solely of $\sigma_{NS}$ protein were found to protect 20-40 nucleotide RNA fragments of reovirus mRNAs, including 3'termini, from

Ar/24.7.86

nuclease digestion (Stamatos and Gomatos, Proc. Natl. Acad. Sci. U.S.A. 79, 3457-3461 [1982]).

The cloning of the reovirus genomic segment encoding $\sigma_{NS}$ protein and its sequencing has been described (Imai et al., Proc. Natl. Acad. Sci. U.S.A. 80, 373-377 [1983] and Richardson and Furuichi, Nucl. Acids Res. 11, 6399-6408 [1983]).

However, in the absence of a recombinant DNA system capable of expressing the desired $\sigma_{NS}$ protein it has not heretofore been possible to produce enough of the protein to fully explore the biological properties of this compound nor was it possible to determine whether the non-specific binding property of ssRNAs was retained by a recombinant produced analog of this protein.

The present invention relates to the genetic engineering of the reovirus genomic segment coding $\sigma_{NS}$ protein to provide and to insert the coding region for $\sigma_{NS}$ protein into an expression plasmid, transforming a suitable microbial host organism with such resulting recombinant expression plasmid, fermenting such transformed microbial host organisms under conditions which favor expression of recombinant $\sigma_{NS}$ protein in such transformed microbial host organisms, purifying the so produced recombinant $\sigma_{NS}$ protein free from microbial protein and employing such recombinant $\sigma_{NS}$ protein in the stabilization of ssRNAs and for the concentration of ssRNA samples for use in hybridization probe assays, preferably supported by covalent attachment to a suitable solid support matrix. As used herein the term "reovirus" is meant to include all three reovirus serotypes designated Types 1, 2 and 3. Type 3 represents a preferred embodiment for use in the present invention.

The present invention may be more readily understood by reference to the following figures; wherein

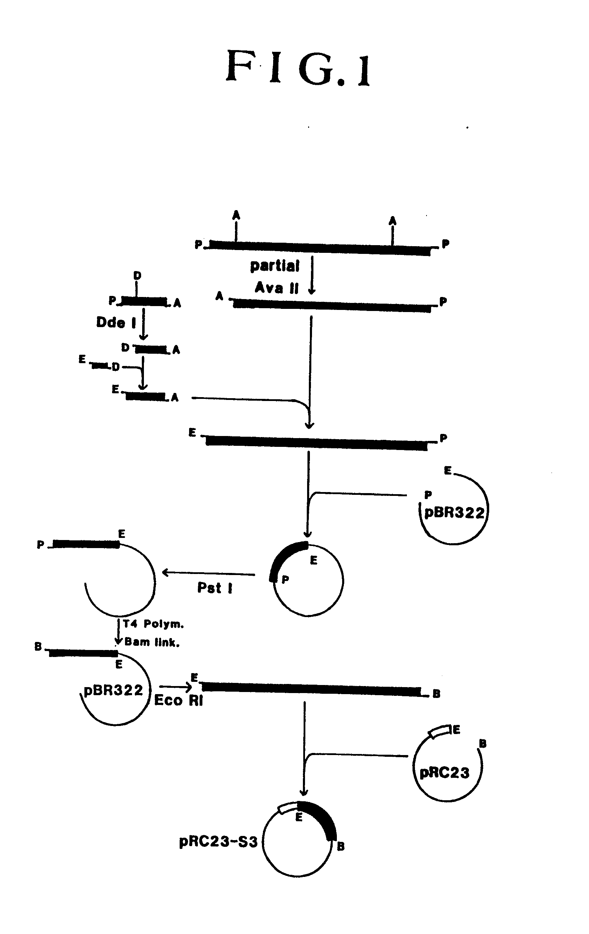

Fig. 1 illustrates the construction of $\sigma_{NS}$ expression plasmid. In essence, the 5' non-coding region of the S3 gene was replaced by an EcoRI site, thus allowing the juxtaposition of the initiating AUG codon for $\sigma_{NS}$ protein with the consensus Shine/Dalgarno sequence present in pRC23. A = AvaII site, B = BamHI site, D = DdeI site, E = EcoRI site, P = PstI site, T4 Polym. = T4 DNA polymerase. The open box depicted on pRC23 represents the $\lambda$ $P_L$ promoter and consensus Shine/Dalgarno ribosomal binding site.

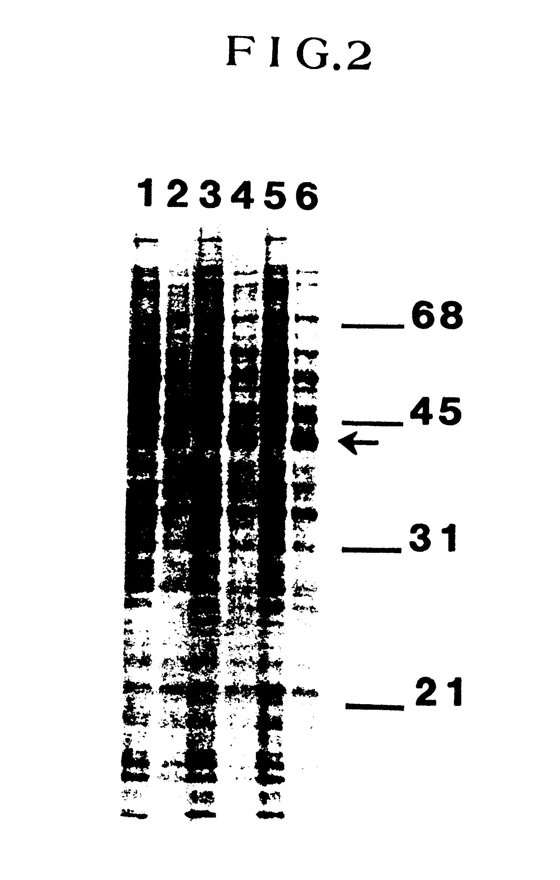

Fig. 2 shows the synthesis of $r\sigma_{NS}$ protein in E.coli. Overnight cultures of E.coli RR1 (pRK 248cIts) transformed with plasmid pRC23-S3 were used to inoculate M9 minimal media containing ampicillin at 100 μg/ml and supplemented with proline (100 μg/ml) and leucine (50 μg/ml). Cultured cells were pulse-labeled, pelleted, dissolved in sample buffer and the pattern of protein synthesis analyzed by 10% SDS-polyacrylamide gel electrophoresis and autoradiography. Lanes 1, 3 and 5, 30°C controls for the three transformants used in this experiment; lanes 2, 4 and 6, 42°C induced cultures of the three transformants. The arrow shows the position of $r\sigma_{NS}$ protein. The MW markers (Bio-Rad Laboratories) were bovine serum albumin (68,000), ovalbumin (45,000), carbonic anhydrase (31,000), and soybean trypsin inhibitor (21,000).

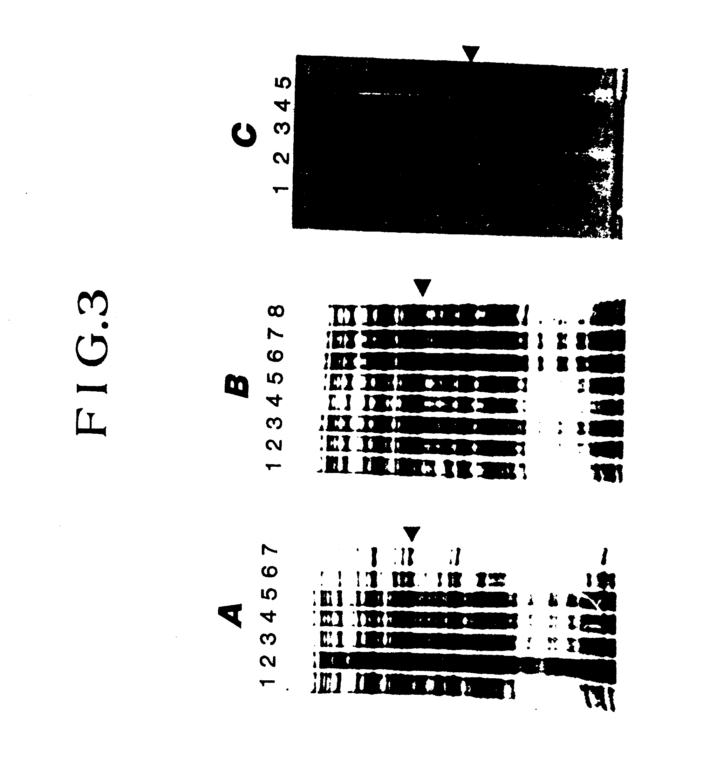

Fig. 3 shows the characteristics of $r\sigma_{NS}$ protein production in E.coli.
(A) Time course of production. A culture of RRI (pRK248 cIts) cells containing pRC23-S3 was grown as described in the text. Aliquots were pulse-labeled and analyzed as described in the legend of Fig. 2. Lane 1, 30°C control; lanes 2-7, 42°C induced culture at 10, 30, 60, 120, 240 and 360 min, respectively, after transfer. The arrow head indicates the position of $r\sigma_{NS}$ protein.

(B) Stability of $r\sigma_{NS}$ protein. Cultures were grown and induced as described above. Thirty min after transfer to 42°C cells were pulse-labeled with $[^{35}S]$-methinonine and then chased with cold methionine. Aliquots (150 µl) were taken at 0, 10, 30, 60, 120, 240 and 360 min after labeling (lanes 2-8, respectively). Lane 1 shows a 30°C control.

(C) Accumulation of $r\sigma_{NS}$ protein in E.coli. Cultures were grown at 30°C in M9 minimal medium supplemented with Casamino acids (CAA) (instead of proline and leucine) to $OD_{600}$ of 0.2-0.3, and then transferred to 42°C. Samples (150 µl) were taken at 1, 2, 3 and 6 hr after transfer (lanes 2-5, respectively), subjected to 10% SDS-polyacrylamide gel electrophoresis and stained with Coomassie Brilliant Blue. Lane 1 is a 30°C control.

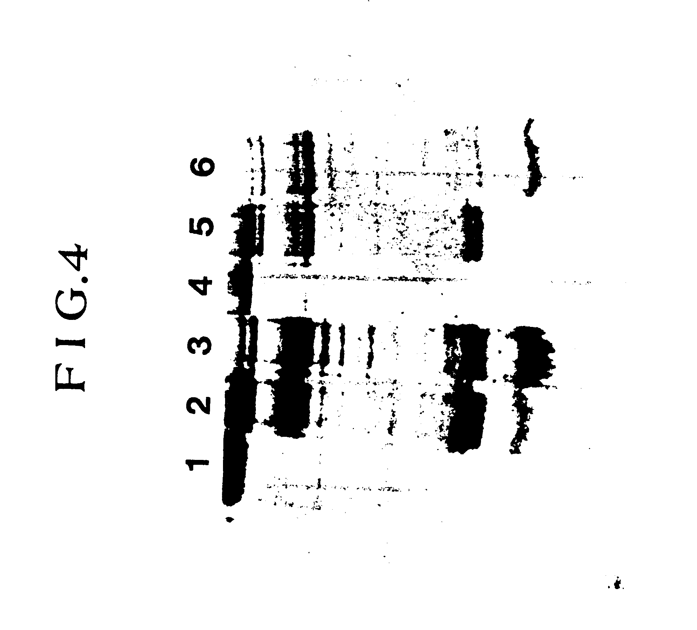

Fig. 4 shows partial V8 protease digestions of $r\sigma_{NS}$ protein and $\sigma_{NS}$ protein from reovirus infected L-cells. Recombinant $\sigma_{NS}$ protein and $\sigma_{NS}$ protein from infected L-cells were partially digested with S.aureus V8 protease and the products analyzed on a 15% SDS-polyacrylamide gel. Digestions were for 30 min at 37°C. Lane 1, undigested $r\sigma_{NS}$ protein; lanes 2 and 3, $r\sigma_{NS}$ protein digested with V8 protease at 5 and 50 µg/ml, respectively; lane 4, undigested $\sigma_{NS}$ protein from infected L-cells; lanes 5 and 6, $\sigma_{NS}$ protein from infected L-cells digested with V8 protease at 5 and 50 µg/ml, respectively.

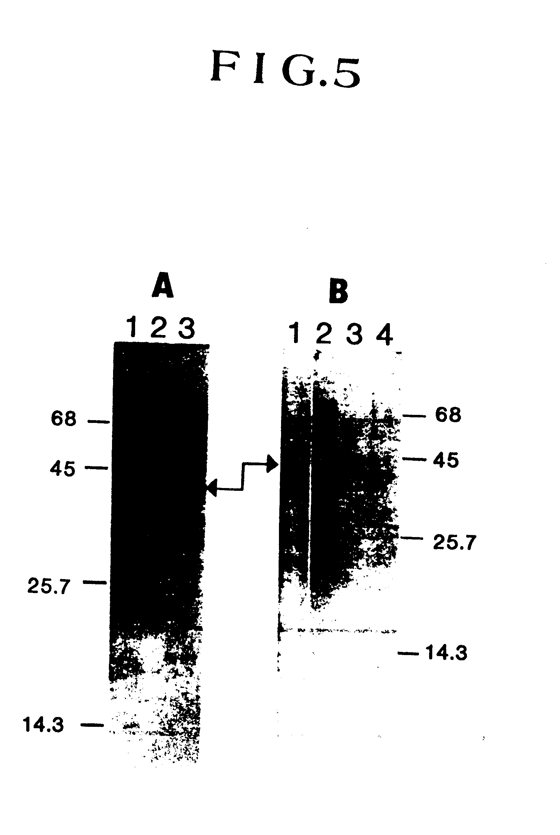

Fig. 5 shows an immunoplot analysis of purified $r\sigma_{NS}$ protein. Poly(A)-agarose column purified $r\sigma_{NS}$ protein was electrophoresed on 12.5% SDS-polyacrylamide gels and then electroblotted onto nitrocellulose filters. The filters were treated to detect proteins recognized by either antibodies raised against $r\sigma_{NS}$ protein (A), or antibodies raised against reovirus infected cells (B). A. Lane 1, BRL prestained MW markers; lane 2, column purified $r\sigma_{NS}$ protein; lane 3, reovirus infected L-cell extracts. B. Lane

- 5 -

0222332

1, column purified $r\sigma_{NS}$ protein; lane 2, reovirus infected L-cell extracts; lane 3, uninfected L-cell extracts;
lane 4, reovirus; the positions of the BRL prestained MW
markers are indicated. The MW markers were bovine serum
albumin (68,000), ovalbumin (45,000), α-chymotrypsinogen
(25,700) and lysozyme (14,300), respectively.

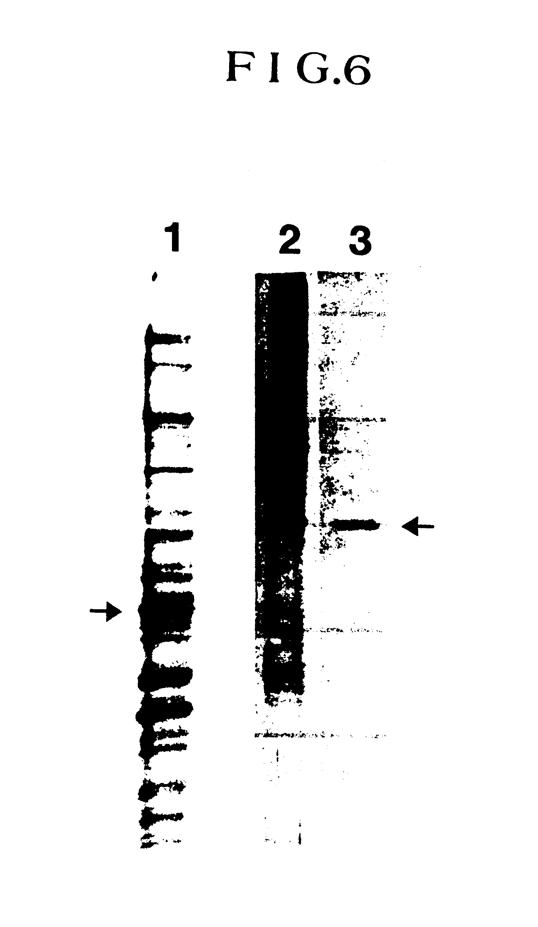

Fig. 6 shows SDS-polyacrylamide gel electrophoresis of
purified $r\sigma_{NS}$ protein. Rebombinant $\sigma_{NS}$ protein was
purified as described in the next. Samples taken at stages
during the purification procedure were subjected to
SDS-polyacrylamide gel electrophoresis and stained with
either Coomassie Brilliand Blue or silver nitrate. Lane 1,
Coomassie Brilliant Blue stained 17,000 rpm supernatant on a
10% SDS-polyacrylamide gel; lane 2, silver nitrate stained
$r\sigma_{NS}$ protein fraction prior to poly(A)-agarose column
chromatography; lane 3, silver nitrate stained poly(A)-
-agarose column purified $r\sigma_{NS}$ protein. Lanes 2 and 3 are
from 12.5% SDS-polyacrylamide gels. The arrows indicate the
position of $r\sigma_{NS}$ protein.

A wide variety of known host-expression plasmid combinations may be employed in the practice of the present invention, such as, for example, plasmids from E. coli including
Col El, pCR, pBR 322 and their derivatives, wider host range
plasmids, e.g. RP4, phage DNA, e.g. the numerous derivatives
of phage lambda, e.g. NM 989 and vectors derived from combination of plasmids and phage DNAs such as plasmids which
have been modified to employ phage DNA or other expression
central sequences or yeast plasmids such as the 2 μ
plasmid or derivatives thereof.

Useful hosts may include bacterial hosts such E. coli
RR1, E. coli W 3110, E. coli HB 101, E. coli X 1776, E. coli
X 2282, E. coli MRCl and strains of Pseudomonas, Bacillus
subtilis, Bacillus stearothermophilus and many other
bacilli, yeasts and other fungi hosts. It is also within the skill of the art to employ as recombinant hosts mammalian, insect or plant cells in culture. It is to be understood that not all host/vector combinations may be equally efficient for purposes of the present invention, a preferred recombinant host/vector system comprises the pRC23 expression plasmid containing the lambda $P_L$ promoter and a consensus Shine/Dalgarno ribosomal binding site and E. coli strain RR1 (ATCC No. 31343). In a further preferred embodiment of the invention pRC23 was propagated in the presence of the low-copy number compatible plasmid pRK248 cIts which carries the gene for a temperature-sensitive lambda cI repressor.

The known expression plasmid pRC23, (for construction see Lacal et al., Proc. Natl. Acad. Sci. U.S.A. $\underline{81}$, 5305--5309 [1984] and European Patent Application No. 99084, published on January 25, 1984) a derivative of pBR322, was used to produce the reovirus nonstructural protein $\sigma_{NS}$ in E.coli. Expression of heterologous genes in pRC23 is driven by the phage lambda $P_L$ promoter. The plasmid was designed to express inserted genes utilizing their own initiating AUG codons, thus allowing the production of protein with a primary amino acid sequence identical to that of the authentic polypeptide. The $P_L$ promoter is under the control of the temperature-sensitive cI repressor encoded by the compatible plasmid pRK248cIts which is described by Bernard and Helinski in Methods Enzymol. $\underline{68}$, 482-492 (1979) and which is deposited and publicly available from the American Type Culture Collection (ATCC), its Accession No. being ATCC 33766. Hence, expression of the heterologous gene is repressed during cell growth at 30°C and induced by shifting cells to 42°C.

The recombinant plasmid pRC23-S3 was constructed following the strategy outlined below. Essentially, a cloned cDNA copy of reovirus genomic segment S3 (encoding $\sigma_{NS}$ protein) was modified by replacing the 5' non-coding region

with an EcoRI cohesive end. This was accomplished by making use of a conveniently located DdeI site and a pair of synthetic complementary deoxyoligonucleotides. To enable correct insertion into plasmid pRC23, a BamHI site was added to the 3' end. Ligation to EcoRI/BamHI digested pRC23 resulted in the initiating AUG for $\sigma_{NS}$ protein being located immediately downstream of the consensus ribosomal binding site engineered into pRC23 as described by Lacal et al, supra.

The strategy employed is outlined in Fig. 1. Initially, a cloned cDNA copy of reovirus type 3 genomic segment S3 derived as described by Imai et al., supra was partially digested with AvaII. After polyacrylamide gel fractionation the purified fragment representing the 5' end of the S3 mRNA (147 bp, including GC tail), was further digested with DdeI, which cut 10 bases downstream of the initiating AUG triplet. Restoration of these bases to the coding region, and the addition of an EcoRI cohesive end, was achieved by ligation to a pair of synthetic complementary deoxyoligonucleotides, prepared by procedures known in the art (see Beaucage and Caruthers, Tetrahydron Lett. 22, 1859-1862 [1981] and Matteucci and Caruthers, J. Amer. Chem. Soc. 103, 3185-3191 [1981]). Following further polyacrylamide gel purification, the fragment containing the modified 5' end (now with an EcoRI site immediately upstream of the initiating AUG codon) was ligated back to the fragment representing the 3' end of the S3 mRNA (1,110 bp, including CG tail), and then the reconstructed S3 gene was subcloned into pBR322 at the EcoRI/PstI sites. Following PstI digestion, T4 DNA polymerase was used to blunt end the PstI site, BamHI linkers added and the resulting EcoRI/BamHI fragment put into pRC23 at the EcoRI/BamHI sites. This expression plasmid was designated pRC23-S3. It is, of course, well known in the art to substitute other linkers specifically designed to provide other restriction sites for insertion into any derived plasmid. All ligations, restriction enzyme digestions and bacterial transformations were carried out using standard conditions essentially as described by Maniatis et al., Molecular Cloning, A laboratory manual, Cold Spring Harbor Laboratory, Cold Spring Harbor N.Y. (1982).

E. coli RRI (pRK248cIts) cells containing the pRC23-S3 expression plasmid were grown overnight at 30°C in L-broth containing ampicillin (100µg/ml) and tetracycline (15µg/ml). These cultures were used to inoculate M9 minimal medium containing ampicillin at 100µg/ml and supplemented with either proline (100µl/ml) and leucine (50µg/ml), or Casamino acids (CAA). Cultures were grown at 30°C to early logarithmic phase ($OD_{600}$=0.2-0.3), divided in half and either maintained at 30°C or transferred to a water bath at 42°C. Recombinant $\sigma_{NS}$ ($r\sigma_{NS}$) protein synthesis was detected by pulse-labeling with [$^{35}$S]--methionine (15-16µCi per 150µl of culture, 1400 Ci/mmole) in M9 minimal medium containing proline and leucine. Synthesis could also be detected by Coomassie Brilliant Blue staining if cultures were grown in M9 minimal medium supplemented with CAA. Cell samples (usually 150µl) were pelleted, dissolved in sample buffer and electrophoresed on either 10% or 12.5% SDS-polyacrylamide gels according to Laemmli, Nature (London) 227, 680-685 (1970).

RRI (pRK248cIts) cells containing pRC23-S3 expression plasmid were grown at 30°C in M9 minimal medium supplemented with CAA to $OD_{600}$ of 0.2-0.3. Cultures were transferred to 42°C and cells collected by centrifugation (Sorval RC3, 3500 rpm, 20 min) 3 hr later. Cell pellets were washed with 25 mM Tris (pH 7.5) containing 5 mM EDTA, repelleted and stored at -20°C. Cells were lysed by resuspension in lysis buffer [25 mM Tris (pH 7.5), 1 mM EDTA, 0.15 M NaCl, 1 mM dithiothreitol (DTT), 1 mM phenylmethylsulphonylfloride (pmsf), 5% glycerol and lysozyme at 0.2 mg/ml] using at least 70 ml per 250 ml of original culture. Chromosomal DNA and cell debris were pelleted by centrifugation at 17,000 rpm for 1 hr in an SW27 rotor. The supernatant was subjected to centrifugation (24,000 rpm, 6 hr, SW27 rotor) onto a 40% (w/v) sucrose cushion. The pellet, containing predominantly $r\sigma_{NS}$ protein, was resuspended in lysis buffer (without lysozyme), made 0.8 M with respect to NaCl and centrifuged at 35,000 rpm for 5 hr in an SW41 rotor. The salt concentration of the supernatant, containing $r\sigma_{NS}$ protein, was reduced to less than 25 mM by buffer exchange with TEM buffer (25 mM Tris (pH 7.5), containing 1 mM 2-mercapto-ethanol) in an Amicon ultrafiltration cell (Amicon Corp., Danvers, MA; YM10 membrane). The supernatant was then applied to a poly(A)--agarose column (Pharmacia, AG-poly(A) type 6 column, size 0.6cm X 3cm), washed extensively with 0.1 M NaCl-TEM buffer followed by 0.2 M NaCl-TEM buffer, and the bound $r\sigma_{NS}$ protein eluted with 0.5 M NaCl-TEM. The eluted fraction, after buffer exchange (to 25 mM Tris (pH 7.5), 1 mM EDTA, 1 mM DTT, 30% glycerol) and concentration in the Amicon ultrafiltration cell, was used in all nucleic acid binding experiments.

Synthesis of $r\sigma_{NS}$ protein in RR1 (pRK248cIts) cells transformed with pRC23-S3 was detected by pulse-labeling with [$^{35}$S]-methionine followed by SDS-polyacrylamide gel electrophoresis and autoradiography (Fig. 2). In the three transformants examined, incubation at 42°C resulted in the synthesis of a protein of approximate molecular weight 41,000 (lanes 2, 4 and 6). The induced protein comigrated with $\sigma_{NS}$ protein synthesized in reovirus infected L-cells (see Fig. 5) and was designated here recombinant $\sigma_{NS}$ protein ($r\sigma_{NS}$ protein). Recombinant $\sigma_{NS}$ protein was detected within 5 min of shifting cultures to 42°C and was still being synthesized 6 hr after induction (Fig. 3A). In a pulse-chase experiment, $r\sigma_{NS}$ protein labeled 30 min after cultures were transferred to 42°C and then chased with cold methionine (3 mg/ml) proved to be quite stable, still being detectable 6 hr after labeling (Fig. 3B). As might be expected from these results, the presence

of $r\sigma_{NS}$ protein in E. coli extracts could be detected by staining SDS-polyacrylamide gels with Coomassie Brilliant Blue (Fig. 3C). Maximum accumulation of $r\sigma_{NS}$ occurred by 3 hr post-induction, at which time $r\sigma_{NS}$ protein constituted 6-7% of total cellular protein. This time was chosen for large scale production of $r\sigma_{NS}$ protein.

The authenticity of $r\sigma_{NS}$ protein produced in E. coli cells was examined in two ways; by 1) partial proteolytic digestions using S. aureus V8 protease; and 2) reactivity with antibody in immunoblots. V8 protease digestions of $r\sigma_{NS}$ protein and authentic $\sigma_{NS}$ protein obtained from reovirus-infected L-cells indicated that the two proteins were, by this criteria, identical (Fig. 4). In immunoblots, rabbit antibodies raised against $r\sigma_{NS}$ protein reacted with authentic $\sigma_{NS}$ protein synthesized in infected L-cells (Fig. 5A). Also, rabbit antibodies raised against reovirus infected cells (and, hence, recognizing most reovirus specific proteins) reacted with $r\sigma_{NS}$ protein (Fig. 5B). These results provided further evidence that $r\sigma_{NS}$ protein was similar, if not identical, to the authentic $\sigma_{NS}$ protein produced in virus-infected cells.

The $r\sigma_{NS}$ protein produced in transformed recombinant cells as described above can be purified as follows. Cells containing the expression plasmid pRC23-S3 were grown and induced in M9 minimal medium containing CAA as described above. The supernatant (lane 1, Fig. 6) was subjected to further centrifugation at 24,000 rpm to pellet $r\sigma_{NS}$ protein. Recombinant $\sigma_{NS}$ protein was solubilized by resuspension in high salt buffer and insoluble contaminants pelleted by centrifugation at 35,000 rpm. The supernatant (lane 2, Fig. 6), after buffer exchange to lower the salt concentration, was subjected to poly(A)-agarose column chromatography as described above. Recombinant $\sigma_{NS}$ protein was purified by this procedure to virtual homogeneity as judged by SDS-polyacrylamide gel electrophoresis and silver staining (lane 3, Fig. 6). This fraction was used for subsequent binding experiments.

$[^{32}P]$-labeled ss and ds nucleic acids, in either TE (25 mM Tris (pH 7.5) containing 1 mM EDTA), or 0.1 M NaCl-TE, were mixed with $r\sigma_{NS}$. After 15 min at 4°C, reactions were diluted 8-fold with the same buffer, and 100 μl duplicate samples were filtered through nitrocellulose filters using a 96 well minifold filtration unit (Schleicher and Schuell, Inc., Keene, NH). Filters were washed with 15 vol (1.5 ml) of 0.4 M NaCl-TE, dried, suspended in Beckman non-aqueous Scintillation fluid and counted in a Beckman LS7800 liquid scintillation spectrometer. Typically, 130-750 ng of $r\sigma_{NS}$ protein and 1-10 ng of nucleic acid were used in binding experiments. Unless indicated, the amount of nucleic acid used in experiments did not saturate the available binding sites on $r\sigma_{NS}$ protein.

Initially the ability of $r\sigma_{NS}$ protein to bind reovirus mRNAs that were methylated and capped was compared to its ability to bind uncapped mRNAs. Accordingly, $[^{32}P]$-labeled reovirus (ss) RNAs synthesized in vitro in either the presence or absence of S-adenosyl methionine (SAM) were mixed with purified $r\sigma_{NS}$ protein and the binding capabilities were determined. Recombinant $\sigma_{NS}$ protein bound both mRNAs with equal efficiency, with greater than 90% of the $[^{32}P]$-labeled input mRNAs being retained on the filter (Table 1), suggesting that 5'-cap structure was not necessary for binding. A similarly purified fraction from non-induced E. coli cultures did not bind either of the mRNAs. Neither did the fraction from induced cultures that eluted with low salt from the poly(A)-agarose column, even though at least 50% of $r\sigma_{NS}$ protein loaded on the column was found in this fraction (Table 1). This result indicated that not all $r\sigma_{NS}$ proteins were capable of binding (ss) RNAs, a characteristic also reported for $\sigma_{NS}$ protein obtained from reovirus-infected L-cells. It should be pointed out

that native $\sigma_{NS}$ protein was essential for binding, as boiling $r\sigma_{NS}$ protein for 5 minutes drastically reduced binding activity (by 90%, data not included). Hence, a non-specific aggregation of $r\sigma_{NS}$ protein and reovirus mRNAs appears to be ruled out.

The specificity of $r\sigma_{NS}$ protein binding was examined next. Recombinant $\sigma_{NS}$ protein was mixed, as described above, with either reovirus ssRNA, cytoplasmic polyhedrosis virus (CPV) ssRNA, reovirus dsRNA or CPV dsRNA. Neither of the $[^{32}P]$-labeled dsRNAs were retained on the filter (Table 2), demonstrating that $r\sigma_{NS}$ protein, like authentic $\sigma_{NS}$ protein from infected L-cells, had low or no affinity for dsRNAs. Both ssRNAs were bound, apparently with equal efficiency (Table 2). This study was extended by examining the ability of $r\sigma_{NS}$ protein to bind yeast rRNA, yeast tRNA and native and denatured dsDNA (a pBR322 fragment). While the overall percentage of input nucleic acid retained on filters was less than that in previous experiments, the results were the same (Table 2). Thus, the three ssRNAs (reovirus, CPV and yeast rRNA) were bound to approximately the same extent whereas the two dsRNAs (reovirus and CPV) were not bound at all. While dsDNA was not retained on filters, consistent with results obtained with the previously characterized $\sigma_{NS}$ protein from infected L-cells, denatured dsDNA was retained, although not to the same extent as ssRNAs. Retention of yeast tRNA, which has extensive secondary structure, was low (Table 2).

The number of available binding sites was estimated in competition experiments using unlabeled reovirus ssRNAs. For approximately 130 ng of $r\sigma_{NS}$ protein, 10-15 ng of competing ssRNA was sufficient to cause a significant reduction in the binding of $[^{32}P]$-labeled reovirus ssRNA (data not shown). From this it could be estimated that the molar ratio of $r\sigma_{NS}$ protein to bound ssRNA was approximately 150 to 1. This was close to that estimated for $\sigma_{NS}$ protein from

infected L-cells by Stamatos and Gomatos, supra.

In a preliminary experiment it was noticed that the capacity of $r\sigma_{NS}$ protein to bind ssRNAs was abolished if all four ribonucleotides were included in the reaction mixture. The ability of each of the four ribonucleotides (ATP, CTP, GTP, and UTP) to inhibit binding was then examined. GTP alone proved able to reduce binding to the extent observed when all four ribonucleotides were included (Table 3). With the amount of $r\sigma_{NS}$ protein used in these experiments (130 ng per reaction) GTP inhibition was evident between 0.5 mM and 1mM (Table 3). The other three ribonucleotides caused little interference with binding even at concentrations of 10 mM (Table 3). This was an important observation for developing $\sigma_{NS}$ protein as a concentrator of ssRNA in test samples to be used in hybridization probe assays. The ability to reversibly bind the ssRNA to $\sigma_{NS}$ protein gives the tester the option of hybridizing the bound ssRNA with the probe or if the bound ssRNA does not hybridize with the probe due to blockage of the hybridization site by the binding of the $\sigma_{NS}$ protein, then the concentrated sample ssRNA can be eluted from the protein complex by use of a solution of GTP or salt. The isolated ssRNA can then be treated with the hybridization probe in a conventional manner. Obviously since the binding of the $\sigma_{NS}$ protein with the ssRNA is non-specific, the specificity of the assay is fully determined by the specificity of the hybridization probe for the desired RNA being assayed.

While it is possible to utilize $\sigma_{NS}$ protein in solution to treat ssRNA to effect stabilization or even to effect concentration of the ssRNA by complexing with $\sigma_{NS}$ protein followed by isolation of the complex such as by sucrose gradient centrifugation the preferred embodiment of the invention is to immobilize the $\sigma_{NS}$ protein on a solid matrix and pass the ssRNA sample solution through the immobilized $\sigma_{NS}$ protein containing matrix. Suitable matrices for this purpose are well known in the art and include Sepharose or Agarose beads treated in a manner known per se to allow direct covalent coupling with protein such as, for example, with cyanogen bromide. Other supports for this purpose include cellulose, particularly nitrocellulose preferably in the form of filters, silica derivatized to react with protein functional groups i.e., -COOH, -OH -NH$_2$ or -SH groups, polymeric microtiter plates and test tubes and the like. Covalent coupling can be achieved using reagents which are conventionally used to react with proteins such as carbodimides e.g. dicyclohexylcarbodimide to provide direct coupling to the support or by using difunctional linking groups such as glutaraldehyde to provide coupling through a linker.

The conditions employed are those well known in the art to effect the reaction between the specific solid matrix employed and the $\sigma_{NS}$ protein depending on the choice of coupling reagent and, where employed, the bifunctional linking group.

It is also possible to bind protein to solid supports, particularly polymeric multititer plates, by utilizing absorption rather than covalent bonding. In such embodiments dilute solutions of the $\sigma_{NS}$ protein in buffer at a pH in the range of about 7-8 are placed in the wells and allowed to dry overnight under a nitrogen stream at 37°C. The resulting plates can be used in the same manner as those prepared using the covalent coupling procedures described above.

The hybridization assay can be carried out conveniently by passing the test solution containing the ssRNA over the solid supported $\sigma_{NS}$ protein if a bead matrix is used such as for example in the form of a chromatographic column. Alternatively, the $\sigma_{NS}$ protein can be used to coat a microtiter plate or the inside of a test tube in a manner known per se. The test sample is then added to the plate or tube and any ssRNA in the sample is allowed an opportunity to complex with the bound $\sigma_{NS}$ protein and then the sample solution is removed and the plate or tube washed. Similarly if a nitrocellulose filter supported $\sigma_{NS}$ protein is used then the test sample is passed through the filter one or more times to allow binding to take place.

The source of test sample can be tissue extracts, body fluids, cell lysates and the like. Assays can be carried out for determining the presence of abnormal genes, to detect genetic abnormalities, to detect active oncogenes, to determine risk of neoplastic disease states, to detect parasitic diseases, to detect infectious diseases or for any other purposes to which hybridization probe assays are run in the art.

The hybridization probe is usually a strand of natural or synthetic DNA or RNA of known sequence which is complementary to at least a portion of the ssRNA which is being assayed. The probe carries a detectable signal ligand to allow detection of the hybridized complex after excess non-reacted probe is washed away. Suitable signal ligands for this purpose are known to the art and many are articles of commerce. Such ligands include radioactive isotopes such as $^{125}I$, $^{32}P$, $^{3}H$, $^{35}S$ and the like, such ligands can be detected in a conventional manner in a scintillation counter; chromophores which can be detected by spectrographic analysis; fluorophors which can be detected by a fluorimeter, enzyme labels such as horseradish peroxidase which can be detected by changes in a chromogenic substrate; and luminescent ligands such as luminol and isoluminol or bioluminescent ligands, such as aequonn which can be detected by a luminometer or any conventional photon counting device.

As pointed out previously the reaction between the hybridization probe and the ssRNA can be carried out directly on the solid support complexed material if the interaction with the $\sigma_{NS}$ protein does not block the hybridization binding site. On the other hand if it is not possible to carry out such a direct assay then the complexed ssRNA is removed from the solid supported $\sigma_{NS}$ protein by treating with a solution of GTP.

The assays are usually read in a (+)/(-) configuration, that is either the indicated RNA sequence is present as seen by the observation of the detectable signal in the monitoring equipment discussed above. However, in appropriate circumstances it is possible to use known concentrations of the test ssRNA in the assay procedure and plotting the values of the signals observed to provide a standard curve which would allow one if the sensitivity of the assay is appropriate, to quantitate the unknown ssRNA.

In a further aspect of the invention a test kit is provided which provides a convenient means for carrying out a hybridization probe assay with concentration of the test sample ssRNA. Such test kit, in a preferred embodiment, comprises a first container containing sufficient $\sigma_{NS}$ protein covalently bound to a solid matrix to carry out multiple assays. As a general rule of thumb the molar ratio of $\sigma_{NS}$ protein to bound ssRNA is approximately 150 to 1 and thus the amount of matrix $\sigma_{NS}$ protein to be used for any single assay determination can be readily determined. The solid matrix can be beads, microtiter plates, tubes or filter paper as indicated previously.

The second container in the kit will contain sufficient hybridization probe reagent bearing a detectable signal ligand to carry out multiple assays. Multiple containers of such reagents with different probes may be provided to allow assays to be carried out against multiple products.

Optionally, the kit may also contain a container of GTP or salt solution, preferably at a concentration of between about 0.5 mM to 1.0 mM, to allow the complexed ssRNA to be eluted free of the $\sigma_{NS}$ protein before the assay is carried out where the complex interferes with hybridization. Again sufficient solution may be provided to allow multiple assays to be carried out.

In further embodiment of the invention, the $\sigma_{NS}$ protein can be provided covalently bound to polymeric microbeads pre-packed in disposable columns allowing the user to simply pass the test solution through the column followed by a wash and then passing the hybridization probe reagent through the column to provide a quick, simple, sensitive and efficient assay system. If the sample contained any specific ssRNA, the probe reagent would hybridize to it and the complex would be detected by the label on the probe reagents.

## Table 1

Binding of reovirus ssRNAs by $r\sigma_{NS}$ protein [a]

|  |  | 42°C induced cultures poly(A)-agarose eluted [b] | | 30°C non-induced cultures poly(A)-agarose eluted [b] |
|  |  | high Salt fraction | low Salt fraction | high Salt fraction |
|---|---|---|---|---|
| % of [$^{32}$P-]-labeled reovirus ssRNAs retained on nitrocellulose membrane | + SAM | 94 | 0 | 0 |
|  | - SAM | 100 | 1 | 0 |

[a] Conditions used as described in the text

[b] Obtained as described in the text.

## Table 2

Specificity of nucleic acid binding to rσ[NS] protein [a]

| | nucleic acid | | | | | | | |
|---|---|---|---|---|---|---|---|---|
| | reovirus ssRNA | CPV ssRNA | reovirus dsRNA | CPV dsRNA | rRNA | tRNA | denatured dsDNA [b] | dsDNA |
| % of [$^{32}$P]-labeled | | | | | | | | |
| nucleic acid    EXP.1 | 78 | 87 | 1 | 2 | | | | |
| retained on | | | | | | | | |
| nitrocellulose EXP.2 | 50 | 79 | 2 | 2 | 51 | 15 | 27 | 1 |
| membrane | | | | | | | | |

[a] Conditions used as described in the text.

[b] dsDNA was denatured by boiling in TE buffer for 5 min and quick chilling in ice $H_2O$.

## Table 3

Inhibition of binding of reovirus ssRNA to r$\sigma_{NS}$ protein by GTP [a]

| | | Ribonucleotide | | | | | |
|---|---|---|---|---|---|---|---|
| | | None | ATP 2mM | CTP 2mM | GTP 2mM | UTP 2mM | Mixture of ATP, CTP, GTP, UTP 2mM each |
| **EXP.1** | | | | | | | |
| % of [$^{32}$P]-labeled reovirus ssRNA retained on | | 100 | 61 | 58 | 5 | 59 | 5 |
| nitrocellulose membrane [b] | | None | GTP | | | | ATP GTP UTP |
| | | | 0.1mM | 0.5mM | 1mM | 2mM  10mM | 10mM 10mM 10mM |
| **EXP.2** | | 100 | 93 | 95 | 29 | 15    0 | 95   81   68 |

[a] Conditions used as described in the text.

[b] Expressed as percentage of control.

WHAT IS CLAIMED IS:

1. A $\sigma_{NS}$ protein composition comprising $\sigma_{NS}$ protein essentially free of other reovirus protein covalently bound to a solid support matrix.

2. The composition of claim 1 wherein said $\sigma_{NS}$ protein is recombinant $\sigma_{NS}$ protein.

3. The composition of claim 1 wherein said solid support matrix is selected from beads, microtiter plates, test tubes or nitrocellulose filter paper.

4. The composition of claim 3 wherein said solid support matrix is beads which are packed within disposable columns.

5. A method for carrying out a hybridization probe assay for a specific ssRNA which method comprises in combination:

   (a)  treating a test sample solution with $\sigma_{NS}$ protein so as to bind ssRNA contained in said test sample non-specifically to said $\sigma_{NS}$ protein;
   (b)  separating said ssRNA-$\sigma_{NS}$ protein complex from the remainder of said test sample so as to concentrate said ssRNA;
   (c)  treating said ssRNA-$\sigma_{NS}$ protein complex with a hybridization probe reagent containing a detectable signal ligand said reagent comprising an RNA or DNA segment complementary to the sequence of the specific ssRNA to be assayed;
   (d)  separating unbound hybridization reagent; and
   (e)  testing the ssRNA-$\sigma_{NS}$ protein complex for the presence of detectable label which indicates the presence of specific ssRNA.

6. The method of claim 5 wherein said ssRNA-$\sigma_{NS}$ protein complex is treated with a GTP or salt solution to dissociate the complex prior to treatment with the said hybridization probe reagent.

7. The method of claim 5 wherein said $\sigma_{NS}$ protein is provided covalently bound to a solid support matrix.

8. The method of claim 5 wherein said $\sigma_{NS}$ protein is recombinant $\sigma_{NS}$ protein.

9. The method of claim 5 wherein said detectable signal ligand is selected from a radioisotope, a chromophore, a fluorophor or an enzyme.

10. A recombinant expression plasmid comprising the gene coding for mature reovirus nonstructural protein $\sigma_{NS}$ operatively connected to a consensus Shine/Dalgarno ribosomal binding site and a promoter sequence.

11. The expression plasmid of claim 10 wherein said promoter is a lambda $P_L$ promoter.

12. The expression plasmid of claim 10 wherein said gene encoding for mature reovirus nonstructural $\sigma_{NS}$ protein is derived from reovirus type 3 genomic segment S3 by partial digestion with Ava II, further digestion with DdeI and ligation of a synthetic nucleotide representing the 10 bases downstream of the initiating AUG triplet lost in the DdeI digestion.

13. A microbial host transformed with the plasmid of claim 10 and capable of expression of $\sigma_{NS}$ protein when said promoter is depressed.

14. The host of claim 13 which has been propagated in the presence of the low-copy-number compatible plasmid pRK 248

cIts carrying the gene for a temperature-sensitive lambda cI represser.

15. The transformed host of claim 13 wherein the host is selected from the group consisting of strains of E. coli, Pseudomonas, Bacillus subtilis, Bacillus stearothermophilus, other bacilli, yeasts, other fungi, animal and plant hosts and human tissue cells.

16. The transformed host of claim 15 which is E. coli.

17. A method for producing recombinant $\sigma_{NS}$ protein comprising the steps of culturing a transformed host of claim 13 under conditions wherein said promoter is derepressed and collecting said $\sigma_{NS}$ protein.

18. The method of claim 17 wherein said $\sigma_{NS}$ protein is purified essentially free of other reovirus and host proteins.

19. The use of a $\sigma_{NS}$ composition as claimed in any of claims 1 to 4 for carrying out a hybridization probe assay for a specific ssRNA.

20. Recombinant $\sigma_{NS}$ protein whenever prepared by a method as claimed in claims 17 or 18.

21. A test kit for carrying out a hybridization probe assay for a specific ssRNA comprising:

(a) a first container containing sufficient $\sigma_{NS}$ protein covalently bound to a solid matrix;
(b) a second container containing sufficient hybridization probe reagent bearing a detectable signal ligand; and
(c) optionally a third container containing GTP or a salt solution.

***

F I G. 1

0222332

RAN 4100/59

0222332

# F I G.2

FIG.3

# FIG.4

0222332

# F I G.5

0222332

# F I G.6